# EUROPÄISCHE PATENTANMELDUNG

(11) **EP 1 903 023 A1**
(43) Veröffentlichungstag der Anmeldung: **26.03.2008**
(21) Anmeldenummer: 07015162.6
(22) Anmeldetag: 02.08.2007
(51) Int. Cl.: C07C 41/24, C07C 209/10, C07C 231/12, C07C 211/56, C07C 217/92, C07D 211/14, C07D 213/75

(54) **Verfahren zur Herstellung von Arylaminen oder Arylamiden**

(30) Priorität: 10.08.2006 DE 102006037399
(71) Anmelder: Archimica GmbH, 65929 Frankfurt am Main (DE)
(72) Erfinder: Jung, Jörg, Dr., 65439 Flörsheim (DE); Meudt, Andreas, Dr., 65719 Hofheim (DE); Lehnemann, Bernd Wilhelm, Dr., 60594 Frankfurt am Main (DE)
(74) Vertreter: Schweitzer, Klaus

(57) **Zusammenfassung**

Die Erfindung betrifft ein Verfahren zur Herstellung von Aryl- oder Heteroarylaminen oder von Aryl- oder Heteroarylamiden durch Kreuzkupplung von primären oder sekundären Aminen oder Amiden mit substituierten Aryl- oder Heteroarylverbindungen, in Gegenwart einer Brønsted-Base und eines Katalysators oder Präkatalysators, wobei der Katalysator
a) ein Übergangsmetall, einen Komplex, ein Salz oder eine Verbindung dieses Übergangsmetalls aus der Gruppe {Ni, Pd} und
b) mindestens einen Liganden aus der Gruppe der bidentaten Bis(phosphino)alkandiyle mit der nachstehenden allgemeinen Formel in einem Lösungsmittel oder Lösungsmittelgemisch enthält, 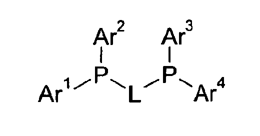 wobei die Reste Ar¹⁻⁴ unabhängig voneinander für Aryl- oder Heteroaryl-Substituenten aus der Gruppe {Phenyl, Naphthyl, Pyridyl und Biphenyl } stehen, bei denen gegebenenfalls Wasserstoff durch niedrig-Alkylsubstituenten, Halogenatome, Sulfonsäuregruppen, Carbonsäuregruppen, niedrig-Alkyloxysubstituenten ersetzt ist, oder Ar¹⁻⁴ für Wasserstoff, C₁-, C₂-Alkyl, geradkettiges, verzweigtes oder cyclisches C₃-C₈-Alkyl steht, welches gegebenenfalls ein oder mehrfach substituiert ist mit Cl, Br, I, OH, NH₂, NO₂, CN, COOH, niedrig-Alkyl-amin, niedrig-Alkyl-diamin, niedrig-Alkyl-Oxy oder niedrig-Alkyl-oxycarbonyl oder niedrig-Alkyl-carbonyloxy, wobei niedrig-Alkyl ein C₁-C₄-Alkylrest bedeutet, und
L für eine Alkandiylbrücke mit 1 bis 20 Kohlenstoffatomen steht, die entweder linear oder verzweigt ist

## Beschreibung

Aryl- bzw. heteroarylsubstituierte Alkyl-/Aryl-amine oder -amide mit verschiedenen Resten am Stickstoff sind bedeutsame und äußerst vielseitig verwendbare Zwischenprodukte in der organischen Synthese, vor allem wenn weitere funktionelle Gruppen im Molekül anwesend sind. Die Bedeutung in der modernen organischen Synthese wird nur durch Limitierungen der Zugänglichkeit dieser Verbindungsklasse eingeschränkt.

Standardverfahren zur Herstellung von aryl- bzw. heteroarylsubstituierten Alkyl-/ Arylaminen ist die *Goldberg*-Reaktion, wobei die Reaktion meist sehr hohe Temperaturen benötigt, um vollständig abzulaufen. Jedoch tolerieren diese in der Regel drastischen Reaktionsbedingungen selten funktionelle Gruppen und reaktive Heteroaromaten und sind nicht oder nur sehr schlecht anwendbar auf elektronenarme Aromaten und zusätzlich nur schwierig zu kontrollieren.

Standardverfahren zur Herstellung von aryl- bzw. heteroarylsubstituierten Alkyl-/ Arylamiden gehen meist aus von den Aminen, die oft aus Nitroverbindungen durch Reduktion hergestellt werden. Dieser Zugang ist aber oft aus sicherheitstechnischen Gründen oder Selektivitätsgründen für Anwendungen im industriellen Maßstab gerade für Heteroaromaten wie etwa Pyridine versperrt.

Somit weisen die derzeit bekannten Verfahren zur Herstellung von aryl- bzw. heteroarylsubstituierten Alkyl-/Aryl-aminen oder -amiden allesamt noch verfahrenstechnische oder wirtschaftliche Nachteile auf, die die Anwendungsbreite zum Teil erheblich einschränken. Von besonderer Bedeutung ist vor allem der hohe Preis der verwendeten Liganden und die oftmals schlechte Zugänglichkeit von größeren Ligand-Mengen, die die Verwendung in industriellen Anwendungen erschwert oder unmöglich macht. Außerdem lassen sich viele Liganden, besonders aus der Klasse der Trialkylphoshine, nur unter striktem Luftausschluß handhaben, um die oxidative Zersetzung oder gar Selbstentzündung zu vermeiden, was die Anwendbarkeit im größeren Maßstab deutlich schwieriger und teurer macht.

Es wäre sehr wünschenswert, ein Verfahren zu haben, das substituierte Alkyl- bzw. Arylamine oder -Amide und Halogenaromaten bzw. Halogenheteroaromaten in die entsprechenden aryl- bzw. heteroarylsubstituierten Alkyl-/Arylamine oder -Amide überführen kann, dabei gleichzeitig hohe Ausbeuten erzielt und mit billigen und einfach zugänglichen und einfach zu handhabenden Liganden auskommt. Wie bereits erwähnt, lösen die bisher dafür veröffentlichten Syntheseverfahren dieses Problem nicht zufriedenstellend, wie anhand von einigen Beispielen weiter demonstriert werden soll:
• Verwendung teurer Liganden (z.B. P^{t}Bu₃, Hartwig et al., US-A 6,100,398) sowie aufwendige Isolierung des Produktes durch Chromatographie
• Verwendung von Luft-empfindlichen Liganden (z.B. P^{t}Bu₃, Hartwig et al., US-A 6,100,398)
• Verwendung von Liganden, die schwierig zu synthetisieren sind (Ferrocenbasierte Liganden, Hartwig et al., WO 0211883)
• aufwendige Isolierung des Produktes durch Chromatographie
• aufwendige bzw. schwierige, oft vielstufige Ligandensynthesen (Buchwald et al., WO 0002887), aufwendige Isolierung des Produktes durch Chromatographie
• Deaktivierung der Metall-Ligand-Komplexe während der Reaktion, oft keine Möglichkeit des Wiederanspringens durch Zugabe weiteren Katalysators und dadurch bedingt Verlust ganzer Ansätze. Dieses Phänomen kann durch zu geringe Stabilität der Komplexe aus Metall und *einzähnigen* Liganden sowie durch die hohe Oxidationsempfindlichkeit der Liganden erklärt werden.

Das vorliegende Verfahren löst alle diese Probleme und betrifft ein Verfahren zur Herstellung von Aryl- und Heteroarylaminen, aryl- bzw. heteroarylsubstituierten Alkyl-/Aryl-amiden durch Kreuzkupplung von primären oder sekundären Alkyl- bzw.

Arylaminen oder von primären oder sekundären Alkyl- bzw. Arylamiden mit substituierten Aryl- oder Heteroarylverbindungen (I), in Gegenwart einer Brønsted-Base und eines Katalysators oder Präkatalysators enthaltend
a.) ein Übergangsmetall, einen Komplex, Salz oder eine Verbindung dieses Übergangsmetalls aus der Gruppe {Ni, Pd}, und
b.) mindestens eines Liganden aus der Gruppe der bidentaten Bis(phosphino)alkandiyle mit der allgemeinen Formel:

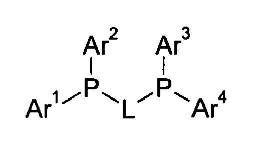

Die Reste Ar¹⁻⁴ stehen unabhängig voneinander für Aryl- oder Heteroaryl-Substituenten aus der Gruppe {Phenyl, Naphthyl, Pyridyl, Biphenyl und ähnliche}, bei denen gegebenenfalls Wasserstoff durch andere Reste wie niedrig-Alkylsubstituenten, Halogenatome, Sulfonsäuregruppen, Carbonsäuregruppen, niedrig-Alkyloxysubstituenten oder ähnliche ersetzt ist, oder Ar¹⁻⁴ steht für Wasserstoff, C₁-, C₂-Alkyl, geradkettiges, verzweigtes oder cyclisches C₃-C₈-Alkyl, welches gegebenenfalls ein oder mehrfach substituiert ist mit Cl, Br, I, OH, NH₂, NO₂, CN, COOH, niedrig-Alkyl-amin, niedrig-Alkyl-diamin, niedrig-Alkyl-Oxy oder niedrig-Alkyl-oxycarbony oder niedrig-Alkyl-carbonyloxy, wobei hier wie im folgenden niedrig-Alkyl ein C₁-C₄-Alkylrest, bevorzugt Methyl oder Ethyl bedeutet.

L steht für eine Alkandiylbrücke mit 1 bis 20 Kohlenstoffatomen, die entweder linear oder verzweigt sein kann. Bevorzugt handelt es sich bei L um eine Alkandiylbrücke aus der Gruppe {Ethan-1,2-diyl, Propan 1,3-diyl, Butan-1,4-diyl oder 2,2-Dimethylpropan-1,3-diyl}.

Einige der o.g. Chelate sind bekannt; sie wurden jedoch bisher überwiegend in C,C-Kupplungen eingesetzt, vor allem in Kupplungen von Grignard-Verbindungen mit Arylhalogeniden unter Katalyse mit derartigen Liganden und Ni-Salzen. Überraschend wurde gefunden, dass die o.g. Liganden in Kombination mit einem Übergangsmetall, einem Komplex, Salz oder einer Verbindung dieses Übergangsmetalls aus der Gruppe {Ni, Pd}, zur katalytischen C,N-Bindungsknüpfung geeignet sind.

Die Reaktion wird typischerweise in einem Lösungsmittel oder Lösungsmittelgemisch durchgeführt. Als Lösungsmittel oder Bestandteil des Lösungsmittelgemisches kann jedes Lösungsmittel verwendet werden, das mit den Reaktanden kompatibel ist, bevorzugt jedoch etherische Lösungsmittel (z. B Dioxan, THF, 1,2-Dimethoxyethan, Monoglyme, Diglyme oder höhere Glymes) oder Aromaten (z. B. Benzol, Toluol, Xylol, Trimethylbenzole, Ethylbenzol) oder Alkohole (Isopropanol, Ethanol, 2-Methoxyethan, 1-Methoxy-2-propanol, Glycol) oder Amide (z. B. DMF, NMP).

Das erfindungsgemäße Verfahren zeichnet sich durch die folgenden Vorteile aus:
• Die Liganden sind kommerziell zu einem wirtschaftlich attraktiven Preis und in großen Mengen erhältlich, insbesondere wenn alle Substituenten Ar¹⁻⁴ Phenylreste sind.
• Ein "Finetuning" der Liganden ist durch eine geeignete Auswahl des Alkandiyl-Linkers relativ leicht möglich. Insbesondere kann der sogenannte "Bite angle" der beiden Phosphin-Untereinheiten so angepaßt werden, daß optimale Reaktivität resultiert. So zeigte sich beispielsweise bei der Synthese von 2,2-Dimethyl-N-pyridin-2-yl-propionamid, dass der "Bite-angle" des Liganden mit L= Propandiyl im Vergleich zu Ethandiyl und Butandiyl offensichtlich ein Optimum darstellt, wie sich durch Vergleich der Ausbeuten feststellen läßt (vgl. Bsp. 10, 11 und 12).
• Neben den attraktiven ökonomischen Eigenschaften zeichnet sich diese Klasse von Liganden durch eine hohe Stabilität gegenüber Luft und Feuchtigkeit aus, so daß das Handling im Gegensatz zu anderen Ligandenklassen deutlich vereinfacht ist, was besonders im technischen Maßstab von größter Wichtigkeit ist.
• Die aus den Übergangsmetallquellen und den bidentaten Liganden resultierenden eigentlich aktiven Komplexe sind sehr stabil, so daß der Katalysator nicht leicht deaktiviert wird und das Katalysatorsystem daher insgesamt sehr robust ist.
• Das erfindungsgemäße Verfahren erweitert durch die genannten, zusätzlich feinjustierbaren Parameter, die Anwendungsbreite der bisher bekannten C,N-Kupplungstechnologien außerordentlich
• Mit dieser Klasse von Liganden können, besonders in heteroaromatischen Systemen, nicht nur Amine, sondern auch Amide gekuppelt werden, was den Anwendungsbereichsbereich stark erweitert, da diese Umsetzung mit anderen Ligandensystemen oft nicht zu bewerkstelligen ist.

Die folgende Gleichung 1 illustriert den Syntheseablauf des erfindungsgemäßen Verfahrens:

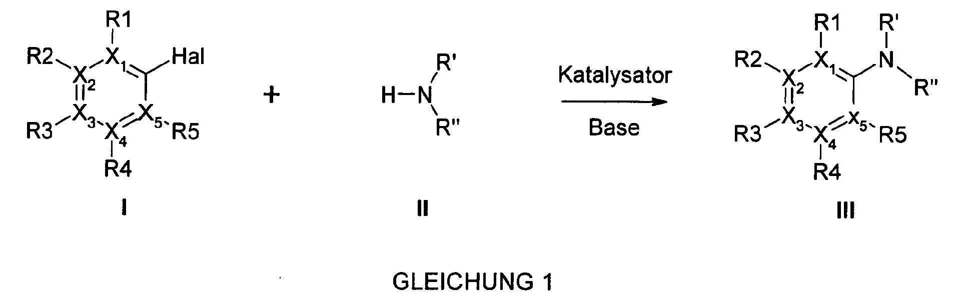

In Gleichung 1 steht Hal für Fluor, Chlor, Brom, Iod, Alkoxy oder für Sulfonat-Abgangsgruppen wie beispielsweise Trifluormethansulfonat (Triflat), Nonafluorbutansulfonat (Nonaflat), Methansulfonat, Benzolsulfonat, para-Toluolsulfonat.

X₁₋₅ bedeuten unabhängig voneinander Kohlenstoff oder XᵢRᵢ (i = Zahl von 1-5) stehen für Stickstoff, oder jeweils zwei benachbarte über eine formale Doppelbindung verbundene XᵢRᵢ stehen gemeinsam für O (Furane), S (Thiophene), NH oder NRᵢ (i = Zahl von 1-5) (Pyrrole).

Bevorzugte Verbindungen der Formel (I), die nach dem erfindungsgemäßen Verfahren umgesetzt werden können, sind z.B. Benzole, Pyridine, Pyrimidine, Pyrazine, Pyridazine, Furane, Thiophene, Pyrrole, beliebig N-substituierte Pyrrole oder Naphthaline, Chinoline, Indole, Benzofurane usw.

Die Reste R₁₋₅ stehen für Substituenten aus der Gruppe {Wasserstoff, Methyl, Ethyl, primäre, sekundäre oder tertiäre, cyclische oder acyclische Alkylreste mit 3 bis 20 C-Atomen, bei denen gegebenenfalls ein oder mehrere Wasserstoffatome durch Fluor oder Chlor oder Brom ersetzt sind, z.B. CF₃, Hydroxy, Alkoxy, Amino, Alkylamino, Dialkylamino, Arylamino, Diarylamino, Alkylarylamino, Pentafluorsulfuranyl, Phenyl, substituiertes Phenyl, Heteroaryl, substituiertes Heteroaryl, Thio, Alkylthio, Arylthio, Diarylphosphino, Dialkylphosphino, Alkylarylphosphino, gegebenenfalls substituiertes Aminocarbonyl, COO⁻, Alkyl- oder Aryloxycarbonyl, Hydroxyalkyl, Alkoxyalkyl, Fluor oder Chlor, Nitro, Cyano, Aryl- oder Alkylsulfon, Aryl- oder Alkylsulfonyl}, oder es können jeweils zwei benachbarte Reste R₁₋₅ zusammen einem aromatischen, heteroaromatischen oder aliphatischen ankondensierten Ring entsprechen.

R' und R" können unabhängig voneinander für gleiche oder unterschiedliche AlkylReste aus der Gruppe {Wasserstoff, C₁-, C₂-Alkyl, geradkettiges, verzweigtes oder cyclisches C₃-C₂₀-Alkyl, substituiertes oder unsubstituiertes Aryl- oder Heteroaryl} oder für gleiche oder unterschiedliche Acyl-Reste aus der Gruppe {Formyl, Acetyl, lineares oder verzweigtes C₃-C₂₀ Acetyl oder substituiertes oder unsubstituiertes Aroyl- oder Heteroaroyl} stehen oder zusammen einen Ring bilden. Bevorzugt bedeuten R' und R" nicht gleichzeitig Wasserstoff.

Typische Beispiele für die Verbindung II sind damit Methyl-, Ethyl-, 1-Methylethyl-, Propyl-, 1-Methylpropyl-, 2-Methylpropyl-, 1,1-Dimethylethyl-, Butyl- und Pentylamin, Cyclopropyl-, Cyclobutyl-, Cyclopentyl-, Cyclohexylamin, Phenyl-, Benzylamin, Morpholin aus der Gruppe der Amine oder Acetamid, Benzamid, 2,2-Dimethylpropionamide aus der Gruppe der Amide.

Als Katalysator wird erfindungsgemäß ein Übergangsmetall oder ein Salz, ein Komplex oder eine metallorganische Verbindung dieses Übergangsmetalls ausgewählt aus der Gruppe {Ni, Pd}, bevorzugt auf einem Träger z.B. Kohle, mit einem bidentaten Bis(phosphino)alkandiyl-Liganden verwendet. Der Katalysator kann in fertiger Form zugesetzt werden oder sich in situ bilden, z.B. aus einem Präkatalysator durch Reduktion oder Hydrolyse oder aus einem Übergangsmetallsalz und zugesetztem Liganden durch Komplexbildung. Der Katalysator wird in Kombination mit einem oder mehreren, mindestens jedoch einem bidentaten Bis(phosphino)alkandiyl-Liganden eingesetzt. Das Übergangsmetall kann in beliebiger Oxidationsstufe eingesetzt werden. Erfindungsgemäß wird es im Verhältnis zum Reaktanden I in Mengen von 0.0001 Mol-% bis 100 Mol-%, bevorzugt zwischen 0.01 und 10 Mol-%, besonders bevorzugt zwischen 0.01 und 2 Mol-% eingesetzt.

Bevorzugt werden Liganden der nachstehend abgebildeten Struktur 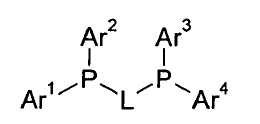 in Verbindung mit Palladium oder Nickel als Katalysator verwendet, worin die Reste Ar¹⁻⁴ unabhängig voneinander für Aryl- oder Heteroaryl-Substituenten aus der Gruppe {Phenyl, Naphthyl, Pyridyl, Biphenyl und ähnliche} stehen, bei denen gegebenenfalls Wasserstoff durch andere Reste wie niedrig-Alkylsubstituenten, Halogenatome, Sulfonsäuregruppen, Carbonsäuregruppen, niedrig-Alkyloxysubstituenten oder ähnliche ersetzt ist.

L hat die weiter oben für die abgebildete Struktur angegebenen Bedeutungen.

Der Zusatz von Brønsted-Basen zum Reaktionsgemisch ist notwendig, um akzeptable Reaktionsgeschwindigkeiten zu erzielen. Als Basen gut geeignet sind z. B. Hydroxide, Alkoholate und Fluoride der Alkali- und Erdalkalimetalle, Carbonate, Hydrogencarbonate und Phosphate der Alkalimetalle und ihre Gemische. Besonders geeignet sind die Basen der Gruppe {Kalium-tert-butylat, Natrium-tert-butylat, Cäsiumtert-butylat, Lithium-tert-butylat sowie die entsprechenden Isopropylate} für die Kupplung von Amiden und die Basen der Gruppe {Natriumcarbonat, Kaliumcarbonatt, Cäsiumcarbonat, Kaliumphosphat} für die Kupplung von Amiden. Dabei wird üblicherweise mindestens die Stoffmenge an Base eingesetzt, die der Stoffmenge des zu kuppelnden Amins oder Amids entspricht, zumeist werden 1.0 bis 6 Äquivalente, vorzugsweise 1.2 bis 3 Äquivalente an Base bezogen auf die Verbindung (II) eingesetzt.

Die Reaktion wird in einem geeigneten Lösungsmittel oder einem ein- oder mehrphasigen Lösungsmittelgemisch durchgeführt, das ein hinreichendes Lösevermögen für alle beteiligten Reaktanden hat, wobei auch heterogene Durchführungen möglich sind (z. B. Einsatz fast unlöslicher Basen). Vorzugsweise wird die Reaktion in polaren, aprotischen oder protischen Lösungsmitteln durchgeführt. Gut geeignet sind offenkettige und cyclische Ether und Diether, Oligo- und Polyether sowie substituierte einfache oder mehrfache Alkohole und gegebenenfalls substituierte Aromaten. Besonders bevorzugt werden ein oder Gemische mehrerer Lösungsmittel der Gruppe {Diglyme, substituierte Glymes, 1,4-Dioxan, Isopropanol, tert-Butanol, 2,2-Dimethyl-1-propanol, Toluol, Xylol} eingesetzt.

Die Reaktion kann bei Temperaturen zwischen Raumtemperatur und dem Siedepunkt des verwendeten Lösungsmittels beim verwendeten Druck durchgeführt werden. Um eine schnellere Reaktion zu erzielen, ist die Durchführung bei erhöhten Temperaturen im Bereich von 0 bis 240°C bevorzugt. Besonders bevorzugt ist der Temperaturbereich von 20 bis 200 °C, insbesondere von 50 bis 150°C.

Die Konzentration der Reaktanden kann in weiten Bereichen variiert werden. Zweckmäßigerweise wird die Reaktion in einer möglichst hohen Konzentration durchgeführt, wobei die Löslichkeiten der Reaktionspartner und Reagentien im jeweiligen Reaktionsmedium beachtet werden müssen. Bevorzugt wird die Reaktion im Bereich zwischen 0.05 und 5 mol/l bezogen auf den im Unterschuss vorliegenden Reaktanden durchgeführt (abhängig von den relativen Preisen der Reaktanden).

Amin oder Amid und aromatischer bzw. heteroaromatischer Reaktionspartner (I) können in Molverhältnissen von 10:1 bis 1:10 eingesetzt werden, bevorzugt sind Verhältnisse von 3:1 bis 1:3 und besonders bevorzugt sind Verhältnisse von 1.2:1 bis 1:1.2.

In einer der bevorzugten Ausführungsformen werden alle Materialien vorgelegt und das Gemisch unter Rühren auf Reaktionstemperatur erhitzt. In einer weiteren bevorzugten Ausführungsform, die sich besonders für die Anwendung im großen Maßstab eignet, wird die Verbindung (II) und gegebenenfalls weitere Reaktanden, z. B. Base und Katalysator oder Präkatalysator während der Reaktion zum Reaktionsgemisch dosiert. Alternativ kann auch durch langsame Zugabe der Base dosiert-kontrolliert durchgeführt werden. Die Selektivitäten sind dabei erfindungsgemäß sehr hoch, es lassen sich meist Bedingungen finden, unter denen bis auf sehr geringe Mengen Dehalogenierungsprodukt keine weiteren Nebenprodukte nachweisbar sind.

Die Aufarbeitung erfolgt üblicherweise - nach Abtrennung von anorganischen Salzen mit Wasser - gemäß den üblichen Verfahren, d. h. im Labor durch Chromatographie und in der Technik durch Destillation oder Umkristallisieren.

Das erfindungsgemäße Verfahren soll durch die nachfolgenden Beispiele erläutert werden, ohne die Erfindung darauf zu beschränken:

### Beispiel 1: Kupplung von 3-Methylpiperidin mit 4-Brombenzotrifluorid (4-Bromtrifluormethylbenzol) (Katalysator: Pd(OAc)₂/2,2-Dimethyl-1,3-bis-(diphenyl phosphino-)propan)

5,8 g Natrium-t-butanolat (60,1 mmol), 5,2 g 3-Methylpiperidin (52,6 mmol) und 8,5 g 4-Brombenzotrifluorid (37,6 mmol) werden in 50 ml Dioxan gelöst bzw. suspendiert und bei 80 °C mit einer Suspension von 0,167 g Palladium(II)-acetat (2 mol%) und 0,43 g 2,2-Dimethyl-1,3-bis(diphenylphosphino)propan (2,5 mol %) versetzt. Anschließend wird am Rückfluß gekocht und der Umsatz per HPLC überwacht. Nach ca. 4 h ist der Umsatz > 98 %. Die Aufarbeitung erfolgt durch Zugabe von Wasser zum Lösen der ausgefallenen Salze, Zugabe von Toluol und Phasentrennung. Die obere, produkthaltige Phase wird einrotiert und das Produkt durch Chromatographie gereinigt. Es konnten so 7,5 g (82 %) Kupplungsprodukt (3-Methyl-1-(4-trifluormethyl-phenyl)-piperidin) erhalten werden.

### Beispiel 2: Kupplung von 3-Methylpiperidin mit 4-Brombenzotrifluorid (Katalysator: Pd(dba)₂/2,2-Dimethyl-1,3-bis(diphenylphosphino)propan)

Wie Beispiel 1, jedoch unter Verwendung von 0,40 g Bis(dibenzylidenaceton)-palladium(0) statt 0,167 g Palladium(II)-acetat. Wie schon im Beispiel 1 war die Umsetzung nach kurzer Reaktionszeit abgeschlossen (in diesem Fall Kochen über Nacht). Ausbeute: 7,8 g (84 %)

### Beispiel 3: Kupplung von 3-Methylpiperidin mit 4-Chlorbenzotrifluorid (Katalysator: Pd(OAc)₂/2,2-Dimethyl-1,3-bis(diphenylphosphino)propan)

Wie Beispiel 1, jedoch unter Verwendung von 6,7 g 4-Chlorbenzotrifluorid (37,6 mmol) statt 8,5 g 4-Brombenzotrifluorid (37,6 mmol). Zum Erzielen eines kompletten Umsatzes (> 95 %) mußte bei Verwendung der weniger reaktiven Chlorverbindung etwas länger gekocht werden (60 h). Die Ausbeute war jedoch mit der aus den beiden vorangegangenen Beispielen vergleichbar (7,1 g, 78 %).

### Beispiel 4: Kupplung von 2-Chloranilin mit 4-Bromanisol (Katalysator: Pd(OAc)₂/2,2-Dimethyl-1,3-bis(diphenylphosphino)propan)

5,9 g Natrium-t-butanolat (61,3 mmol), 6,7 g 2-Chloranilin (52,6 mmol) und 7,0 g 4-Bromanisol (37,6 mmol) werden in 50 ml Dioxan gelöst bzw. suspendiert und bei 80 °C mit einer Suspension von 0,167 g Palladium(II)-acetat (2 mol%) und 0,43 g 2,2-Dimethyl-1,3-bis(diphenylphosphino)propan (2,5 mol %) versetzt. Anschließend wird am Rückfluß gekocht und der Umsatz per HPLC überwacht. Der Umsatz ist nach 72 h vollständig (> 95 %). Die Aufarbeitung erfolgt durch Zugabe von Wasser zum Lösen der ausgefallenen Salze, Zugabe von Toluol und Phasentrennung. Die obere, produkthaltige Phase wird einrotiert und das Produkt durch Chromatographie gereinigt. Es konnten so 6,4 g (73 %) Kupplungsprodukt (2-Chlorphenyl)-(4-methoxyphenyl)amin gewonnen werden.

### Beispiel 5: Kupplung von 2-Chloranilin mit 4-Brombenzotrifluorid (Katalysator: Pd(OAc)₂/2,2-Dimethyl-1,3-bis(diphenylphosphino)propan)

4,8 g Natrium-t-butanolat (48,4 mmol), 4,1 g 2-Chloranilin (33,2 mmol) und 7,0 g 4-Brombenzotrifluorid (30,2 mmol) werden in 50 ml Dioxan gelöst bzw. suspendiert und bei 80 °C mit einer Suspension von 0,14 g Palladium(II)-acetat (2 mol%) und 0,33 g 2,2-Dimethyl-1,3-bis(diphenylphosphino)propan (2,5 mol %) versetzt. Anschließend wird am Rückfluß gekocht und der Umsatz per HPLC überwacht. Der Umsatz ist nach 72 h vollständig (> 95 %). Die Aufarbeitung erfolgt durch Zugabe von Wasser zum Lösen der ausgefallenen Salze, Zugabe von Toluol und Phasentrennung. Die obere, produkthaltige Phase wird einrotiert und das Produkt durch Chromatographie gereinigt. Es konnten so 5,3 g (64 %) Kupplungsprodukt (2-Chlorphenyl)-(4-trifluormethylphenyl)amin gewonnen werden.

### Beispiel 6: Kupplung von 3-Methylpiperidin mit 4-Brombenzotrifluorid (Katalysator: Pd(OAc)₂/1,4-bis(diphenylphosphino)butan)

2,9 g Natrium-t-butanolat (30,1 mmol), 2,6 g 3-Methylpiperidin (26,3 mmol) und 4,2 g 4-Brombenzotrifluorid (18,8 mmol) werden in 25 ml Dioxan gelöst bzw. suspendiert und bei 80 °C mit einer Suspension von 0,088 g Palladium(II)-acetat (2 mol%) und 0,201 g 1,4-Bis(diphenylphosphino)butan (2,5 mol %) versetzt. Anschließend wird am Rückfluß gekocht und der Umsatz per HPLC überwacht. Nach ca. 5 h ist der Umsatz > 98 %. Die Aufarbeitung erfolgt durch Zugabe von Wasser zum Lösen der ausgefallenen Salze, Zugabe von Toluol und Phasentrennung. Die obere, produkthaltige Phase wird einrotiert und das Produkt durch Chromatographie gereinigt. Es konnten so 3,1 g (87 %) Kupplungsprodukt (3-Methyl-1-(4-trifluoromethyl-phenyl)-piperidine) erhalten werden.

### Beispiel 7: Kupplung von 3-Methylpiperidin mit 4-Brombenzotrifluorid (Katalysator: Pd(OAc)₂/1,3-Bis(diphenylphosphino)propan)

2,9 g Natrium-t-butanolat (30,1 mmol), 2,6 g 3-Methylpiperidin (26,3 mmol) und 4,2 g 4-Brombenzotrifluorid (18,8 mmol) werden in 25 ml Dioxan gelöst bzw. suspendiert und bei 80 °C mit einer Suspension von 0,088 g Palladium(II)-acetat (2 mol%) und 0,194 g 1,3-Bis(diphenylphosphino)propan (2,5 mol %) versetzt. Anschließend wird am Rückfluß gekocht und der Umsatz per HPLC überwacht. Nach ca. 5 h ist der Umsatz > 98 %. Die Aufarbeitung erfolgt durch Zugabe von Wasser zum Lösen der ausgefallenen Salze, Zugabe von Toluol und Phasentrennung. Die obere, produkthaltige Phase wird einrotiert und das Produkt durch Chromatographie gereinigt. Es konnten so 3,2 g (90 %) Kupplungsprodukt (3-Methyl-1-(4-trifluoromethyl-phenyl)-piperidine) erhalten werden.

### Beispiel 8: Kupplung von 3-Methylpiperidin mit 4-Brombenzotrifluorid (Katalysator: Pd(OAc)₂/1,2-Bis(diphenylphosphino)ethan)

2,9 g Natrium-t-butanolat (30,1 mmol), 2,6 g 3-Methylpiperidin (26,3 mmol) und 4,2 g 4-Brombenzotrifluorid (18,8 mmol) werden in 25 ml Dioxan gelöst bzw. suspendiert und bei 80 °C mit einer Suspension von 0,088 g Palladium(II)-acetat (2 mol%) und 0,187 g 1,2-Bis(diphenylphosphino)ethan (2,5 mol %) versetzt. Anschließend wird am Rückfluß gekocht und der Umsatz per HPLC überwacht. Nach ca. 5 h ist der Umsatz > 98 %. Die Aufarbeitung erfolgt durch Zugabe von Wasser zum Lösen der ausgefallenen Salze, Zugabe von Toluol und Phasentrennung. Die obere, produkthaltige Phase wird einrotiert und das Produkt durch Chromatographie gereinigt. Es konnten so 2,9 g (82 %) Kupplungsprodukt (3-Methyl-1-(4-trifluoromethyl-phenyl)-piperidine) erhalten werden

Neben den Kupplungen von Aminen sind die beschriebenen Systeme vor allem auch aktiv in der Kupplung von Amiden mit Aromaten, d. h. insbesondere in der Kupplung mit Heteroaromaten wie Pyridinen. Bei diesen Kupplungen kann vorteilhaft Kaliumcarbonat als Base verwendet werden.

### Beispiel 9: Kupplung von 2-Chlorpyridin mit 4-Fluorbenzamid (Katalysator: Pd(OAc)₂/2,2-Dimethyl-1,3-bis(diphenylphosphino)propan)

4,0 g Kaliumcarbonat (28,9 mmol), 3,5 g 4-Fluorbenzamid (25,3 mmol) und 2,1 g 2-Chlorpyridin (18,1 mmol) werden in 25 ml Dioxan gelöst bzw. suspendiert und bei 80 °C mit einer Suspension von 0,036 g Palladium(II)-acetat (0,9 mol%) und 0,200 g 1,3-Bis(diphenylphosphino)propan (2,5 mol %) versetzt. Anschließend wird am Rückfluß gekocht und der Umsatz per HPLC überwacht. Nach Kochen über Nacht ist der Umsatz > 98 %. Die Aufarbeitung erfolgt durch Zugabe von Wasser zum Lösen der ausgefallenen Salze, Zugabe von Toluol und Phasentrennung. Die obere, produkthaltige Phase wird einrotiert und das Produkt durch Chromatographie gereinigt. Es konnten so 3,5 g (89 %) Kupplungsprodukt (4-Fluoro-N-pyridin-2-yl-benzamide) erhalten werden.

### Beispiel 10: Kupplung von 2-Chlorpyridin mit 2,2-Dimethylpropionamid (Katalysator: Pd(OAc)₂/2,2-Dimethyl-1,3-bis(diphenylphosphino)propan)

3,1 g Kaliumcarbonat (22,7 mmol), 2,0 g 2,2-Dimethylpropionamid (20,0 mmol) und 1,7 g 2-Chlorpyridin (14,2 mmol) werden in 40 ml Dioxan gelöst bzw. suspendiert und bei 80 °C mit einer Suspension von 0,027 g Palladium(II)-acetat (0,9 mol%) und 0,156 g 2,2-Dimethyl-1,3-bis(diphenylphosphino)propan (2,5 mol %) versetzt. Anschließend wird am Rückfluß gekocht und der Umsatz per HPLC überwacht. Nach Kochen über Nacht ist der Umsatz > 98 %. Die Aufarbeitung erfolgt durch Zugabe von Wasser zum Lösen der ausgefallenen Salze, Zugabe von Toluol und Phasentrennung. Die obere, produkthaltige Phase wird einrotiert und das Produkt durch Chromatographie gereinigt. Es konnten so 2,6 g (88 %) Kupplungsprodukt (2,2-Dimethyl-N-pyridin-2-yl-propionamid) erhalten werden.

### Beispiel 11: Kupplung von 2-Chlorpyridin mit 2,2-Dimethylpropionamid (Katalysator: Pd(OAc)₂/1,4-bis(diphenylphosphino)butan)

Wie Beispiel 10, jedoch wurden statt 0,156 g 2,2-Dimethyl-1,3-bis(diphenylphosphino)-propan (2,5 mol %) 0,151 g 1,4-bis(diphenylphosphino)butan (2,5 mol %) verwendet. Ausbeute 2,4 g (81 %)

### Beispiel 12: Kupplung von 2-Chlorpyridin mit 2,2-Dimethylpropionamid (Katalysator: Pd(OAc)₂/1,2-bis(diphenylphosphino)ethan)

Wie Beispiel 10, jedoch wurden statt 0,141 g 2,2-Dimethyl-1,3-bis(diphenylphosphino)-propan (2,5 mol %) 0,151 g 1,4-bis(diphenylphosphino)ethan (2,5 mol %) verwendet. Ausbeute 2,5 g (85 %)

### Beispiel 13: Kupplung von 2-Chlorpyridin mit 2,2-Dimethylpropionamid (Katalysator: Pd(OAc)₂/Triphenylphosphin; Vergleichsversuch)

Wie Beispiel 10, jedoch wurden statt 0,141 g 2,2-Dimethyl-1,3-bis(diphenylphosphino)-propan (2,5 mol %) 0,187 g Triphenylphosphin (5 mol%) verwendet. Mit diesem Liganden konnte jedoch kein Umsatz erzielt werden.

## Patentansprüche

1. Verfahren zur Herstellung von Aryl- oder Heteroarylaminen oder von Aryl- oder Heteroarylamiden durch Kreuzkupplung von primären oder sekundären Aminen oder Amiden mit substituierten Aryl- oder Heteroarylverbindungen, in Gegenwart einer Brønsted-Base und eines Katalysators oder Präkatalysators, **dadurch gekennzeichnet, dass** der Katalysator
a) ein Übergangsmetall, einen Komplex, ein Salz oder eine Verbindung dieses Übergangsmetalls aus der Gruppe {Ni, Pd} und
b) mindestens einen Liganden aus der Gruppe der bidentaten Bis(phosphino)alkandiyle mit der nachstehenden allgemeinen Formel in einem Lösungsmittel oder Lösungsmittelgemisch enthält, 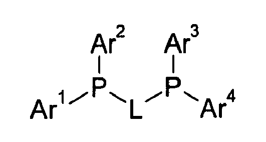
wobei die Reste Ar¹⁻⁴ unabhängig voneinander für Aryl- oder Heteroaryl-Substituenten aus der Gruppe {Phenyl, Naphthyl, Pyridyl und Biphenyl} stehen, bei denen gegebenenfalls Wasserstoff durch niedrig-Alkylsubstituenten, Halogenatome, Sulfonsäuregruppen, Carbonsäuregruppen, niedrig-Alkyloxysubstituenten ersetzt ist,
oder Ar¹⁻⁴ für Wasserstoff, C₁-, C₂-Alkyl, geradkettiges, verzweigtes oder cyclisches C₃-C₈-Alkyl steht, welches gegebenenfalls ein oder mehrfach substituiert ist mit Cl, Br, I, OH, NH₂, NO₂, CN, COOH, niedrig-Alkyl-amin, niedrig-Alkyl-diamin, niedrig-Alkyl-Oxy oder niedrig-Alkyl-oxycarbonyl oder niedrig-Alkyl-carbonyloxy, wobei niedrig-Alkyl ein C₁-C₄-Alkylrest bedeutet, und
L für eine Alkandiylbrücke mit 1 bis 20 Kohlenstoffatomen steht, die entweder linear oder verzweigt ist.

2. Verfahren nach Anspruch 1, wobei L eine Alkandiylbrücke aus der Gruppe {Ethan-1,2-diyl, Propan 1,3-diyl, Butan-1,4-diyl oder 2,2-Dimethylpropan-1,3-diyl} ist.

3. Verfahren nach Anspruch 1 oder 2, **dadurch gekennzeichnet, dass** die substituierte Aryl- oder Heteroarylverbindung eine Verbindung der Formel (I) ist, 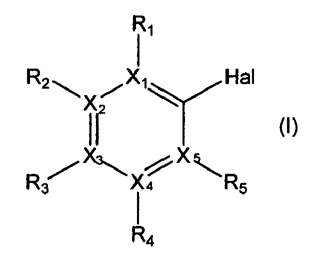 Hal für Fluor, Chlor, Brom, Iod, C₁-C₄-Alkoxy, Trifluormethansulfonat, Nonafluortrimethyl-methansulfonat, Methansulfonat, 4-Toluolsulfonat, Benzolsulfonat, 2-Naphthalinsulfonat, 3-Nitrobenzolsulfonat, 4-Nitrobenzolsulfonat, 4-Chlorbenzolsulfonat oder 2,4,6-Triisopropylbenzolsulfonat steht, und
R₁₋₅ für gleiche oder verschiedene Substituenten aus der Gruppe {Wasserstoff, Methyl, Ethyl, primäre, sekundäre oder tertiäre, cyclische oder acyclische Alkylreste mit 3 bis 20 C-Atomen, bei denen gegebenenfalls ein oder mehrere Wasserstoffatome durch Fluor oder Chlor oder Brom ersetzt sind, Hydroxy, niedrig-Alkyloxy, Amino, niedrig-Alkylamino, Di-niedrig-alkylamino, Arylamino, Diarylamino, niedrig-Alkylarylamino, Pentafluorsulfuranyl, Phenyl, substituiertes Phenyl, Heteroaryl, substituiertes Heteroaryl, Thio, niedrig-Alkylthio, Arylthio, Diarylphosphino, Di-niedrigalkylphosphino, niedrig-Alkylarylphosphino, gegebenenfalls substituiertes Aminocarbonyl, CO₂⁻, niedrig-Alkyl-oder Aryloxycarbonyl, Hydroxy-niedrig-alkyl, niedrig-Alkyloxy-niedrig-alkyl, Fluor, Chlor, Nitro, Cyano, Aryl- oder niedrig-Alkylsulfon, Aryloder niedrig-Alkylsulfonyl, wobei niedrig-Alkyl ein C₁-C₄-Alkylrestund Aryl Phenyl oder Naphthyl und Heteroaryl Pyridinyl, Imidazolyl, Thienyl oder Furanyl bedeutet}, oder wobei jeweils zwei benachbarte Reste R₁₋₅ zusammen einem aromatischen, heteroaromatischen oder aliphatisch ankondensierten Ring entsprechen.

4. Verfahren nach Anspruch 1, 2 oder 3, **dadurch gekennzeichnet, dass** das primäre oder sekundäre Amin oder Amid eine Verbindung der Formel (II) ist, 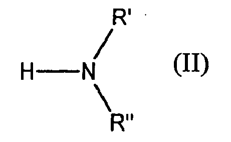 wobei R' und R" unabhängig voneinander für gleiche oder unterschiedliche Reste aus der Gruppe {Wasserstoff, Methyl, Ethyl, lineares, verzweigtes C₃-C₂₀ -Alkyl oder cyclisches C₃-C₂₀-Alkyl, substituiertes oder unsubstituiertes Aryl- oder Heteroaryl, wobei Aryl Phenyl oder Naphthyl und Heteroaryl Pyridinyl, Imidazolyl, Thienyl oder Furanyl bedeutet} stehen oder für gleiche oder unterschiedliche Acyl-Reste aus der Gruppe {Formyl, Acetyl, lineares oder verzweigtes C₃-C₂₀ Acetyl oder substituiertes oder unsubstituiertes Aroyl- oder Heteroaroyl wobei Aroyl Phenylcarbonyl oder Naphthylcarbonyl und Heteroaroyl Pyridinylcarbonyl, Imidazolylcarbonyl, Thienylcarbonyl oder Furanylcarbonyl bedeutet} stehen oder zusammen einen Ring bilden.

5. Verfahren nach einem der Ansprüche 1 bis 4, **dadurch gekennzeichnet, daß** es sich bei dem zur Katalyse verwendeten Übergangsmetall um Palladium handelt.

6. Verfahren nach Anspruch 5, **dadurch gekennzeichnet, daß** die verwendete Palladiumquelle Palladium(II)-acetat ist.

7. Verfahren nach einem der Ansprüche 1 bis 6, **dadurch gekennzeichnet, daß** die Alkandiylbrücke L eine Länge von 1 bis 4 Kohlenstoffatomen hat.

8. Verfahren nach einem der Ansprüche 1 bis 7, **dadurch gekennzeichnet, dass** 1,0 bis 3 Äquivalente an Bronsted-Base bezogen auf die substituierte Aryl- oder Heteroarylverbindung eingesetzt werden.

9. Verfahren nach Anspruch 8, **dadurch gekennzeichnet, daß** die verwendete Brønsted-Base Natrium-tert-butanolat ist.

10. Verfahren nach Anspruch 8, **dadurch gekennzeichnet, daß** die verwendete Brønsted-Base Kaliumcarbonat ist.

11. Verfahren nach einem der Ansprüche 1 bis 10, **dadurch gekennzeichnet, daß** die verwendete substituierte Aryl- oder Heteroarylverbindung ein gegebenenfalls zusätzlich substituiertes 2-Halogenpyridin oder ein gegebenenfalls zusätzlich substituiertes 4-Halogenpyridin ist.

12. Verfahren nach einem der Ansprüche 1 bis 11, **dadurch gekennzeichnet, dass** als Lösungsmittel oder Lösungsmittelgemisch Kohlenwasserstoffe, halogenierte Kohlenwasserstoffe, offenkettige oder cyclische Ether oder Diether, Oligo- oder Polyether, tertiäre Amine, DMSO, NMP, DMF, DMAc sowie substituierte einfache oder mehrfache Alkohole oder gegebenenfalls substituierte Aromaten oder ein Gemisch mehrerer dieser Lösungsmittel eingesetzt wird.

13. Verfahren nach einem der Ansprüche 1 bis 12, **dadurch gekennzeichnet, daß** die Kreuzkupplungsreaktion bei einer Temperatur im Bereich von 0 bis 240°C durchgeführt wird.

14. Verfahren nach einem der Ansprüche 1 bis 13, **dadurch gekennzeichnet, daß** der Katalysator im Verhältnis zur substituierten Aryl- oder Heteroarylverbindung in Mengen von 0.001 Mol-% bis 25 Mol-% eingesetzt wird.
